(19) Europäisches Patentamt
European Patent Office
Office européen des brevets

(11) Veröffentlichungsnummer: **0 235 159**
**B1**

(12) **EUROPÄISCHE PATENTSCHRIFT**

(45) Veröffentlichungstag der Patentschrift: 08.08.90

(21) Anmeldenummer: 86900665.0

(22) Anmeldetag: 21.12.85

(86) Internationale Anmeldenummer:
PCT/EP85/00735

(87) Internationale Veröffentlichungsnummer:
WO 86/03967 17.07.86 Gazette 86/17

(51) Int. Cl.⁵: **A 61 K 9/68**, A 23 G 3/30

(54) KAUGUMMI UND VERFAHREN ZU SEINER HERSTELLUNG.

(30) Priorität: 27.12.84 CH 6187/84

(43) Veröffentlichungstag der Anmeldung:
09.09.87 Patentblatt 87/37

(45) Bekanntmachung des Hinweises auf die
Patenterteilung:
08.08.90 Patentblatt 90/32

(84) Benannte Vertragsstaaten:
AT BE CH DE FR GB IT LI LU NL SE

(56) Entgegenhaltungen:
EP-A-0 151 344
AT-C- 350 728
DE-A-2 808 160
LU-A- 74 297
US-A-2 290 120

(73) Patentinhaber: Gergely, Gerhard, Dr.
Gartengasse 8
A-1050 Wien (AT)

(72) Erfinder: GERGELY, Gerhard, Dr.
Gartengasse 8
A-1050 Wien (AT)

(74) Vertreter: Büchel, Kurt F., Dr.
Bergstrasse 297
FL-9495 Triesen (LI)

Courier Press, Leamington Spa, England.

Beschreibung

Die Erfindung betrifft einen Kaugummi, bestehend aus einer granulierten Kaugummibase, sowie Füll-, Zuschlag- und Wirkstoffen; sie betrifft auch ein Verfahren zur Herstellung dieses Kaugummis, bei dem die Kaugummibase unter 0 Grad C abgekühlt und zerkleinert wird.

Der eingangs erwähnte Kaugummi ist bereits mehrfach vorgeschlagen worden (DE—A 2808160, US—A 2,290,120, LU—A 74.297). Alle diese bekannten Vorschläge haben keinen Eingang in die Praxis gefunden, da die einzelnen Kaugummi-Granulatkörner sich beim Kauen nur sehr schlecht zu einer zusammenhängenden Masse verbinden. Im Gegenteil: die Mischung der Granulatkörner mit Zucker und anderen Feststoffen bewirkt erst recht eine Trennung der Granulatkörner, die sich im Mund erst nach dem Weglösen von Zucker und anderen Feststoffen durch intensives Zusammenschieben mit der Zunge verbinden lassen.

Nun wäre aber ein Kaugummi erwünscht, der sofort eine zusammenhängende Masse ergibt, aus der dann der Zucker, Aroma- und/oder Wirkstoffe etc. langsam herausgelöst werden, was insbesondere für bitter schmeckende Substanzen, wie z.B. bestimmte pharmazeutische Wirkstoffe wesentlich ist. Ein solcher Kaugummi wird erfindungsgemäss durch die Massnahmen der Ansprüche 1 bis 3 erzielt. Durch die Einbettung der Kaugummi-Granulatkörner in eine Matrix aus Fetten und/oder Wachsen wird beim Kauen sofort oder zumindest sehr rasch eine zusammenhängende Masse gebildet, insbesondere wenn die Matrix in die Kaugummi-Granulatkörner eindiffundiert ist und deren Oberfläche bereits "weichgemacht" hat.

Die erfindungsgemässen, kaugummihältigen Tabletten sind auch besser hantierbar, lagerbeständig und herstellbar, wenn sie mit einer an sich bekannten Drageechichte überzogen sind.

Die Erfindung hat sich des weiteren zur Aufgabe gestellt, ein Verfahren zur Herstellung—insbesondere pharmazeutischer—Kaugummis zu schaffen, welches die Herstellung auch kleiner Produktchargen ermöglicht und bei dem die dem Kaugummi zuzusetzenden Wirkstoffe exakt dosierbar sind. Die Herstellung von pharmazeutischen Kaugummis auf konventionellen Kaugummimaschinen ist nämlich aus zwei Gründen schwierig: erstens ist der Austoss zu gross, und zweitens lässt sich eine Wirkstoffdosierung und auch eine gleichförmige Herstellung des Kaugummis im pharmazeutischen Sinne schwer erzielen. Darüber hinaus sind die Erfordernisse der GMP (Good Manufacturing Practice) schwer erfüllbar.

Die Herstellung des erfindungsgemässen Kaugummis gelingt jedoch überraschend durch die in den Ansprüchen 4 bis 7 angeführten Massnahmen. Das Verfahren läuft so ab, dass man

(a) eine Kaugummibase auf eine Temperatur unter −20 Grad C abkühlt und zu einer Korngrösse von 0,2 bis 1 mm zerkleinert,

(b) eine Masse bestehend aus Fetten oder Wachsen mit einem Schmelzpunkt von 35 bis 50 Grad C schmilzt, in der resultierenden Schmelze einen Füllstoff suspendiert, die Schmelze abkühlen lässt und bei einer Temperatur unter 0 Grad C zu einer Korngrösse von 0,2 bis 0,5 mm mahlt,

(c) die unter (a) erhaltene granulatförmige Kaugummibase mit der unter (b) erhaltenen granulatförmigen Masse in einem Vakuummischbehälter unter Mischen bei Temperaturen von max. +5 Grad C vereinigt und mit einem mit einer Matrix oder mit der genannten Fett- oder Wachsmasse umhüllten Wirkstoff und mit üblichen Zuschlagstoffen versetzt,

(d) die so erhaltene, auf einer Temperatur von max. +5 Grad C gehaltene Mischung in einer—vorzugsweise gekühlten—Tablettenpresse zu normalen pharmazeutischen Tabletten verpresst und diese bei 10 bis maximal 20 Grad C lagert.

(e) die tablettenförmigen Kaugummikerne durch Dragieren mit einer Deckschicht überzieht, sodann langsam auf 35 bis 60, vorzugsweise 40 bis 45 Grad C erwärmt und schliesslich auf Raumtemperatur abkühlen lässt.

Diese Vorgangsweise ist bevorzugt, obwohl es auch möglich ist, die aus den verschiedenen Granulaten gepressten Tabletten in mit entsprechenden Ausnehmungen und einer Trennbeschichtung versehenen Blechen der Wärmebehandlung zu unterziehen.

Im erfindungsgemässen Verfahren liegen die Kaugummibestandteile als normales Granulat vor, das allerdings bei Temperaturen um 0 Grad C herum verarbeitet werden muss. Zur Herstellung dieses Granulates wird in Stufe (a) der Kaugummi, der normalerweise in Platten als fertige Gummibase geliefert wird, auf −20 Grad C gebracht und in klimatisierten Räumen auf schnellaufenden Mühlen auf die genannte Korngrösse von 0,2 bis 1 mm zerkleinert.

Die Masse bestehend aus Fetten und/oder Wachsen kann in Stufe (b) auf einem mit Rührwerk versehenen Wasserbad geschmolzen werden. In der Schmelze werden übliche Füllstoffe, wie z.B. Aerosil, Sorbitol, Dextrin, usw., suspendiert, welche dazu beitragen, dass man nach dem Abkühlen der Masse auf unter +5 Grad C, vorzugsweise unter 0 Grad C, ein mahlfähiges Gemisch erhält, das auf die genannte Korngrösse von 0,2 bis 0,5 mm zerkleinert wird.

Beispiele von Fetten und/oder Wachsen, die für diese Masse verwendet werden können, sind Mono-, Di- und Triglyceride gesättigter, geradzahliger Fettsäuren der Kettenlänge C10 bis C18, pflanzliche Fette aller Art, Cera alba (Bienenwachs), gehärtetes (hydriertes) Rizinusöl, Polyäthylenglykol, Polypropylenglykol, Butylglykoläther, etc.

Die in Stufe (a) und (b) erhaltenen Granulate werden nach wie vor auf einer Temperatur von max. +5 Grad C, vorzugsweise um 0 Grad C, gehalten, in einen Vakuummischbehälter eingebracht und in diesem durch Mischen bei der genannten Temperatur zu einem aus den beiden